# EUROPEAN PATENT SPECIFICATION

(11) **EP 4 627 997 B1**
(45) Date of publication and mention of the grant of the patent: **09.12.2026**
(21) Application number: 24168589.0
(22) Date of filing: 04.04.2024
(51) Int. Cl.: A61B 5/00, A61B 5/308, A61B 5/316, A61B 5/346, A61B 5/366, A61B 5/367

(54) **METHOD FOR PROCESSING AN ELECTROCARDIOGRAM**
VERFAHREN ZUR VERARBEITUNG EINES ELEKTROKARDIOGRAMMS
PROCÉDÉ DE TRAITEMENT D'UN ÉLECTROCARDIOGRAMME

(43) Date of publication of application: 08.10.2025
(73) Proprietor: Institute of Scientific Instruments of the Czech Academy of Sciences, v. v. i., 61264 Brno (CZ); St. Anne's University Hospital Brno, 60200 Brno (CZ); VDI Technologies s.r.o., 61200 Brno (CZ)
(72) Inventor: Jurak, Pavel, Brno (CZ); Halamek, Josef, Brno (CZ); Viscor, Ivo, Brno (CZ); Plesinger, Filip, Brno (CZ); Vondra, Vlastimil, Brno (CZ); Smisek, Radovan, Kurim (CZ); Leinveber, Pavel, Brno (CZ); Curila, Karol, Praha (CZ); Navratil, Miroslav, Jihlava (CZ)
(74) Representative: Harber IP s.r.o.

(56) References cited:
- US-A1- 2021 161 457
- PAVEL JURAK: "3-Dimensional ventricular electrical activation pattern assessed from a novel high-frequency electrocardiographic imaging technique: principles and clinical importance", vol. 11, no. 1, 1 June 2021 (2021-06-01), US, XP093162681, ISSN: 2045-2322, Retrieved from the Internet <URL:https://www.ncbi.nlm.nih.gov/pmc/articles/PMC8169848/pdf/41598_2021_Article_90963.pdf> [retrieved on 20240515], DOI: 10.1038/s41598-021-90963-4
- JURAK PAVEL ET AL: "Ultra-High-Frequency Electrocardiography", 2021 COMPUTING IN CARDIOLOGY (CINC), CREATIVE COMMONS, vol. 48, 13 September 2021 (2021-09-13), pages 1 - 4, XP033999461, DOI: 10.23919/CINC53138.2021.9662795
- SALES CLARA ET AL: "Left Bundle Branch Area Pacing Generates More Physiological Ventricular Activation Sequences than Right Ventricular Pacing", 2023 COMPUTING IN CARDIOLOGY (CINC), CINC, vol. 50, 1 November 2023 (2023-11-01), pages 1 - 4, XP034506302, DOI: 10.22489/CINC.2023.316
- PAVEL LEINVEBER: "Ultra-high-frequency ECG volumetric and negative derivative epicardial ventricular electrical activation pattern", SCIENTIFIC REPORTS, 7 March 2024 (2024-03-07), XP093163720, Retrieved from the Internet <URL:https://www.nature.com/articles/s41598-024-55789-w.pdf> [retrieved on 20240515]

## Description

### Field of Art

The present invention relates to a method of processing of broad-band multi-channel electrocardiogram (ECG) and to an apparatus for carrying out the method. The results obtained from the processing method allow to determine electrical activation properties of heart ventricles, in particular the location of the electrical activation and/or distance of the heart ventricles from the body surface.

### Background Art

Devices for recording electrical activity of heart, electrocardiogram or ECG, are commonly used in cardiology for heart disease diagnostic. Standard ECG monitors provide an ECG output signal in a frequency range of up to about 100 Hz. High-resolution ECG monitors with a higher sampling rate 1-4 kHz are available on the market to a limited extent. State of art of high-frequency ECG analysis is described in the study of Guy Amit, et al., Journal of Electrocardiology, 2014;47(4):505-511. Morphology of the QRS complex in the band from 150 up to 250 Hz, i. e. centralization, conceivably broadening and bifurcation of amplitude peaks, often defined by means of RAZ (Reduced Amplitude Zone) parameters, serves for diagnostics of pathological phenomena in myocardium, in particular of ischemic heart disease.

Recently, the inventors applied high-frequency electrocardiography (HF-ECG, 150-350 Hz) to compute ventricular electrical delay (VED) determined in 676 left bundle branch block (LBBB) patients - F Plesinger et al. Circulation: Arrhythmia and Electrophysiology. 2018;11:e005719, originally published April 26, 2018, https://doi.org/10.1161/CIRCEP.117.005719. The results showed that VED predicts survival in biventricular resynchronization patients in a more reliable manner than the conventional QRS-derived parameters. A more recent study indicated that VED might be a more useful predictor for cardiac resynchronization therapy response than ECG characteristics of strict LBBB, Halamek J, et al., Plos ONE May 2019, https://doi.org/10.1371/journal.pone.0217097. The concept with a single-band ultra-high-frequency (500-1000 Hz) 12-lead ECG by Jurak P et al., J Interv Card Electrophysiol. 2017; 49(3): 245-254 presented measures of electrical depolarization patterns and ventricular electrical dyssynchrony. Method of measuring and analyzing the ultra-high-frequency signals of myocardial activity and their processing to time numerical parameters that describe the electrical ventricular dyssynchrony was described by the inventors in the U.S. Pat. No. 9,949,655.

In EP 3827743, the inventors provided a method and an apparatus for processing signals obtained from broad-band ultra-high-frequency oscillations generated by myocardium (UHF-ECG), i. e. the ECG components in frequency ranges within the 100-1000 Hz range, allowing to accurately identify time distribution of ventricular depolarization in order to reliably diagnose heart abnormalities and pathologies which can effectively be treated by cardiac pacing. The manuscript "Ultra-high-frequency ECG volumetric and negative derivative epicardial ventricular electrical activation pattern" (https://doi.org/10.1038/s41598-021-90963-4_published 7 March 2024) introduces a novel high-frequency electrocardiographic imaging technique that measures intramural ventricular electrical activation. The high-frequency ECG signals are obtained from at least two ECG channels: amplitude envelopes are computed in at least two frequency ranges, QRS complexes are segmented into envelopes per frequency and lead, for each frequency range and channel a normalized average QRS envelope is formed, and then all the normalised QRS envelopes of all frequency ranges are then averaged within each channel.

The present invention aims to provide a more precise location of the ventricular electrical activation. UHF-ECG methods cannot distinguish the distance of the source of electrical activation. The present invention defines a method and apparatus that can identify nearby and distant sources and thus determine, for example, the activation of the ventricular septum and the free wall of the left or right ventricle. This information is crucial in the clinical evaluation of the ventricular activation pattern and in determining electrical dyssynchrony in both ventricles.

### Summary of the Invention

The present invention provides a method of processing an electrocardiogram, which comprises the following steps:
- measuring the electrocardiogram comprising at least two channels and sensing a separate electrocardiogram signal on each of the at least two channels; wherein the measuring step is carried out by an apparatus comprising at least two sensors measuring the electrocardiogram signal, wherein the outputs of the at least two sensors are connected to an input of one or more analogue amplifiers, and an output of the said one or more analogue amplifiers is connected to an input of one or more analogue signal to digital signal converters, and an output of the one or more analogue signal to digital signal converters is connected to a processing unit, wherein the at least two sensors, the one or more analogue amplifiers, and the one or more analogue signal to digital signal converters have the transmission bandwidth of at least 0.2 kHz,
- and performing the following steps in a processing unit, comprising an input connected to the output of the analogue to digital signal converters and an output connected to at least one imaging unit:
- selecting at least two, preferably non-overlapping, frequency ranges of the signal in each of the said at least two channels;
- calculating an amplitude or power envelope of the signal in each selected frequency range in each channel;
- dividing the calculated envelope of the signal in each frequency range in each channel into QRS complex envelopes, wherein each QRS complex envelope comprises one annotated QRS complex;
- computing, for each of the selected frequency ranges in each channel, an average envelope or a median envelope **(FE)** as an average or a median of the QRS complex envelopes in the frequency range;
- optionally performing baseline correction for each average envelope or each median envelope by subtracting the mean or median value of an interval in which no QRS complex is present, wherein the interval in which no QRS complex is present is an interval between the QRS complexes, to remove noise background;
- normalizing each average or median envelope (FE), optionally after its baseline correction, to obtain a normalized average envelope or a normalized median envelope **(NFE)** in each frequency range of each channel; wherein the normalizing is performed by dividing the average envelope or the median envelope (FE) of the frequency range by its integral or by a maximal value reached in the average envelope or in the median envelope, in each frequency range and each channel separately, while the integral or the maximal value is calculated within an interval of at least 30 ms before the annotation of the QRS complex and at least 30 ms after the annotation of the QRS complex;
- if more than two frequency ranges are selected in each channel, a combined average envelope or a combined median envelope (ANFE) is calculated from normalized average envelopes or normalized median envelopes (NFE) from at least two lowest frequency ranges and/or from at least two highest frequency ranges within each channel, wherein each frequency range is included in only one combined average envelope or combined median envelope (ANFE) and the calculation results in two combined average envelopes or combined median envelopes (ANFEs);
- calculating, for each channel, the difference between the values FE, NFE and/or ANFE in the lower frequency range and the values FE, NFE and/or ANFE in the higher frequency range; wherein
- a positive difference between the values FE, NFE and/or ANFE in the lower frequency range and the values FE, NFE and/or ANFE in the higher frequency range identifies ventricular electrical activation of distant heart ventricular regions; and
- a negative difference between the values FE, NFE and/or ANFE in the lower frequency range and the values FE, NFE and/or ANFE in the higher frequency range identifies ventricular electrical activation of nearby heart ventricular regions

### Detailed description of the Invention

The invention will be further illustrated using exemplary embodiments, and with reference to the figures.

Object of the invention is a method of processing of multichannel broad-band ultra-high-frequency electrocardiogram.

The broad-band multi-channel electrocardiogram is a plurality of signals recorded by a plurality of measurement electrodes and presented as a plurality of signals in channels. The signals are measured in a frequency range above 0.2 Hz. Currently, the signals are typically measured in frequency ranges starting from 100 Hz and up to 1000 Hz, but any measuring frequency range is compliant with the present invention.

Typically, 2 to 256 channels are used. Signals from the leads V1-V6 or V1-V8 are preferred for this invention. The signals are a dependency of electrical potential (voltage) on time.

The step of measuring electrocardiogram is carried out by an apparatus comprising at least two sensors measuring the electrocardiogram signal, wherein the output(s) of at least two sensors is connected to an input of one or more analogue amplifiers, and an output of the said one or more analogue amplifiers is connected to an input of one or more analogue signal to digital signal converters, and an output of the one or more analogue signal to digital signal converters is connected to a processing unit, wherein the at least two sensors, the one or more analogue amplifiers, and the one or more analogue signal to digital signal converters have the transmission bandwidth of at least 0.2 kHz.

In some embodiments, the apparatus contains the same number of sensors, analogue amplifiers and analogue signal to digital signal converters, i.e., for each sensor, there is an analogue amplifier and an analogue signal to digital signal converter.

A "sensor" is an electrode attached to the surface of the human body. "Lead" means the resulting digitized signal assembled according to the standard montages used in cardiology. "Channel" refers to a digitized signal from the sensor or lead.

The method processes electrocardiogram comprising signals from at least two channels. Signals from the leads V1-V6 or V1-V8 are preferred. Signals from all channels, or signals only from some channels can be used in the method of the invention.

At least two, preferably non-overlapping, frequency ranges are selected in each of the said at least two channels. The frequency ranges are frequency bands above the frequency of 0.2 Hz. Width of each frequency range may preferably be from 20 to 1000 Hz. The frequency ranges are preferably the same in each channel. In some preferred embodiments, a lower frequency range may be within 20 to 150 Hz and a higher frequency range may be within 150 to 1000 Hz.

An envelope of the signal is calculated for each frequency range in each channel.

An envelope is a smooth curve outlining the extremes of the oscillating signal. In this invention, an upper envelope is considered as the envelope, i.e., the curve outlining the upper extremes of the signal. The envelope may be an amplitude envelope or a power envelope. The amplitude envelope is an envelope outlining the amplitude extremes of the signal. The power envelope is an envelope outlining the power extremes of the signal (power = amplitude squared).

In preferred embodiments, the amplitude or power envelopes of the ECG channel are calculated using Hilbert transformation, or the amplitude envelopes of the ECG channel are calculated by filtration, conversion of the signal obtained in this way into an absolute value and smoothing it, or the power envelopes of the ECG channel are calculated by filtration, raising the ECG signal to the power of two and smoothing it.

The calculated envelope of the signal in each frequency range in each channel is divided into QRS complex envelopes, wherein a QRS complex envelope is a portion of the envelope of the signal, said portion corresponding to one QRS complex, i.e., outlining one QRS complex. A QRS complex is the combination of three of the graphical deflections shown on an electrocardiogram, wherein the QRS complex corresponds to the depolarization of the right and left ventricles. QRS complex contains the waves Q, R and S. Q and S waves are downward deflections and R is an upward deflection The position of the QRS complex (also called "QRS complex annotation") corresponds approximately to center of the QRS complex. QRS complexes are detected and annotated by known algorithms such as Pan-Tompkins or Hilbert transform algorithms. Many other algorithms are available and known to a person skilled in the art. The annotation algorithms annotate all QRS complexes in one frequency range in the same way and all QRS complexes in all frequency ranges and in all channels in the same way.

QRS complex envelope is preferably a portion of the envelope of the signal which starts at least 50 ms, or 50 to 500 ms, or 50 to 150 ms, or 120 to 200 ms before the annotation of the QRS complex, and ends at least 50 ms, or 50 to 500 ms, or 50 to 150 ms, or 120 to 200 ms after the annotation of the QRS complex.

An average envelope or a median envelope (designated as FE) is then computed from the QRS complex envelopes within each of the frequency ranges, in each of the channels. This step increases a signal-to-noise ratio for each frequency range in each channel.

Baseline correction may optionally be performed for each average envelope or median envelope by subtracting the mean (average) or median value from a temporal interval in which no QRS complex is present, in order to remove noise background. Baseline correction is particularly useful if the integral is used in the following step of normalization. The interval in which no QRS complex is present is an interval anywhere between the S wave of one QRS complex and the Q wave of the following QRS complex.

The average envelope or median envelope are normalized to obtain a normalized average envelope or normalized median envelope (NFE) for each frequency range in each channel. The normalization is performed by dividing the average envelope or the median envelope of each frequency range in each channel by its integral (FEI) or by a maximal value reached in the average envelope or median envelope. The integral or the maximal value is calculated within an interval of a minimum of 50 ms before the QRS complex annotation and a minimum of 50 ms after the QRS complex annotation. One normalized average or median envelope (NFE) is obtained per each frequency range, in each channel.

Calculations of average, median, or normalization are performed in the sequence of points whose time distance from the QRS complex annotation is equal. In other words, each point (e.g., sampling point) of the average, median, or normalized envelope is calculated as an average, median, or normalized value, respectively, of the points in the same temporal position of all envelopes over which the calculation of the average, median or normalization is performed.

If more than two frequency ranges are selected in each channel, a combined average envelope or combined median envelope (ANFE) is calculated from the normalized average envelopes or normalized median envelopes of at least two lowest frequency ranges or at least two highest frequency ranges within each channel. The calculation is performed by averaging or determining the median of normalized average envelopes or normalized median envelopes from the at least two lowest frequency ranges or at least two highest frequency ranges within the channel. This calculation is performed in each channel. As a result, two combined average envelopes or two combined median envelopes are obtained in each channel.

In each channel, the FE, NFE and/or ANFE for the lower frequency range are designated LFE, LNFE, and LANFE, respectively, and the FE, NFE, and ANFE for the higher frequency range are designated HFE, HNFE, and HANFE, respectively.

For each channel, the difference (DIFF) between the values FE, NFE and/or ANFE in the lower frequency range and the values FE, NFE and/or ANFE in the higher frequency range, respectively, is calculated at each time point. The calculation can preferably be described as LFE minus HFE, and/or LNFE minus HNFE, and/or LANFE minus HANFE. Furthermore, differences LNFE minus HANFE, and/or LFE minus HANFE, and/or LFE minus HNFE can also be used.

The lower frequency FE, NFE, or ANFE **(LFE, LNFE,** or **LANFE)** register ventricular electrical activation in both more distant and nearby heart ventricular regions; while the higher frequency envelopes FE, NFE, or ANFE **(HFE, HNFE,** or **HANFE)** register ventricular electrical activation predominately in nearby heart ventricular regions.

The terms "lower", "higher", "lowest", "highest" are used herein to describe the relative positions of frequency ranges within a channel, or to describe values and parameters relating to these frequency ranges.

The terms "distant" and "nearby" in relation to heart ventricular regions have an established meaning in the field of the invention, known to clinicians and ECG specialists. The location of the "distant" and "nearby" depends on the electrode's position. For example: In the case of the lead V1, "distant" means the septum and apex region, while "nearby" means the free wall of the right ventricle. In the case of the lead V3, "distant" means the septum region, while "nearby" means the apex region. In the case of the lead V6, "distant" means the septum and apex region, while "nearby" means the free wall of the left ventricle.

In each channel, a positive difference between LFE, LNFE, or LANFE and HFE, HNFE, or HANFE, preferably between LFE and HFE, LNFE and HNFE, or LANFE and HANFE, identifies ventricular electrical activation of distant heart ventricular regions at the respective time point(s) or time interval(s). A "positive difference" means that the LFE, LNFE, or LANFE is higher than HFE, HNFE, or HANFE.

In each channel, a negative difference between LFE, LNFE, or LANFE and HFE, HNFE, or HANFE, preferably between LFE and HFE, LNFE and HNFE, or LANFE and HANFE identifies ventricular electrical activation of nearby heart ventricular regions at the respective time point(s) or time interval(s). A "negative difference" means that the LFE, LNFE, or LANFE is lower than HFE, HNFE, or HANFE.

In a preferred embodiment, the invention allows to construct a differential ventricular depolarization map **(DVDM).** DVDM is constructed as a matrix wherein each row of differential ventricular depolarization matrix is represented by a difference between LFE, LNFE, or LANFE and HFE, HNFE, or HANFE for one of the said at least two ECG channels, and wherein the columns correspond to time points or time intervals. Then the positive values in each differential ventricular depolarization matrix row are assigned a first colour and negative values in each differential ventricular depolarization matrix row are assigned a second colour. The first colour in DVDM identifies ventricular electrical activation of distant heart ventricular regions at the respective time point(s) or time interval(s). The second colour identifies ventricular electrical activation of nearby heart ventricular regions at the respective time point(s) or time interval(s). The first colour and the second colour may have shades corresponding to the level of the ventricular electrical activation.

In one preferred embodiment, the method of the invention further comprises a step of comparison of an integral **FEI** of the average envelopes or median envelopes **FEI** between channels. The channel with the maximum value **FEImax** shows the body surface location where the heart is closest to the body surface. In a subsequent step, a value **FEIslope** is calculated, wherein FEIslope is defined as the proportion of FEI to FEImax. FEIslope indicates the heart's distance from the body surface. A lower **FEIslope** value means a higher drop in the FEI values relative to the maximal value. This indicates a smaller distance of the heart ventricles from the body surface. FEIslope has values in the range 0-1. Whereas values closer to 1 mean a matching signal intensity in the selected leads and, thus a similar distance of the electrodes from the heart. It is when the heart is far from the body's surface electrodes. Values closer to 0 mean a differential signal intensity in the selected leads and, thus, a differential distance of the electrodes from the heart. It is when the heart is close to the body's surface electrodes.

In one embodiment, the method of the invention further comprises a step of construction of a spatial activation map **SAM,** wherein the spatial activation map is constructed as a projection of the differential ventricular depolarization map to geometrical interpretation of the ventricles. DVDM distinguishes near and far ventricular segment activations by color, each color has an associated time and location on body surface. The first colour identifies ventricular electrical activation of the distant ventricular septal region in all V1-V8 leads, and the second colour identifies ventricular electrical activation of the nearby ventricular regions, in leads V1-V2 of the free wall of the right ventricle, in leads V3-V4 of the apical region, and in leads V5-V8 of the free wall of the left ventricle. The first colour and the second colour may have shades corresponding to the level of the ventricular electrical activation.

### Brief Description of Drawings

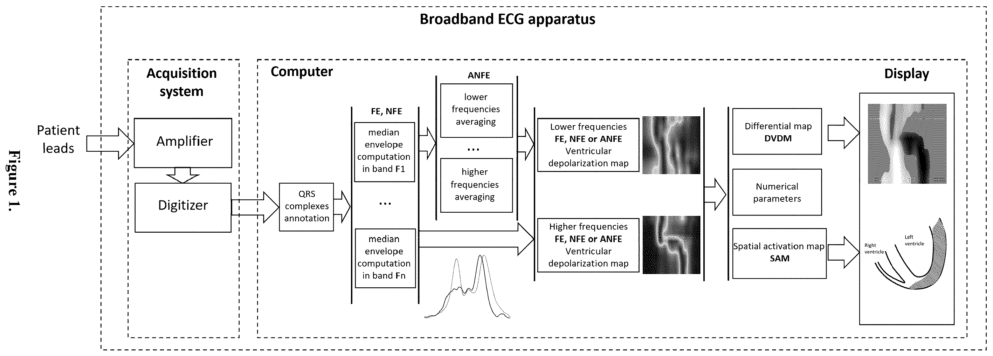

**Figure 1** schematically shows a block diagram of an apparatus suitable for carrying out the method of the invention. The schematic block diagram includes an amplifier and a digitizer for recording the ECG signal from the human body. Further blocks indicate processing of the signal and visualizing the result according to the method of the invention.

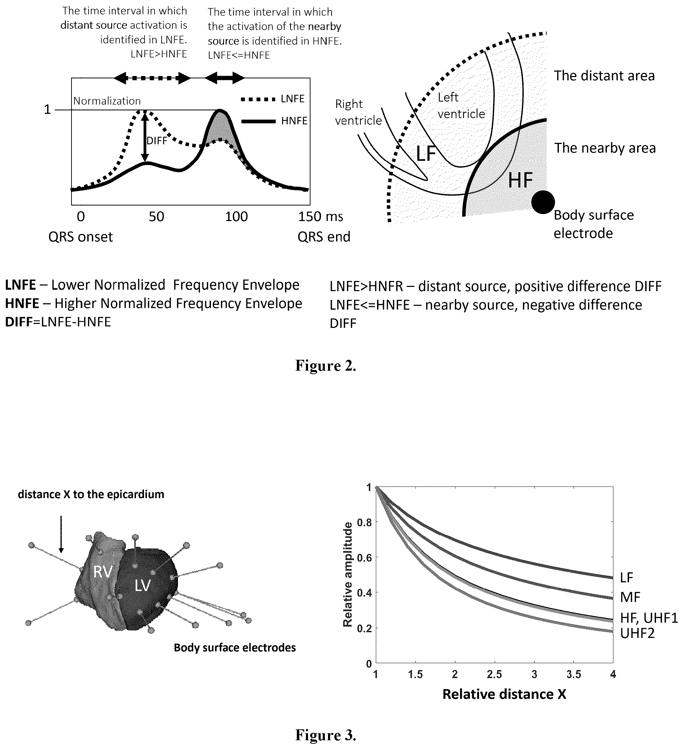

**Figure 2** shows the determination of the difference DIFF from LNFE and HNFE envelopes and its interpretation. Higher frequency (HF) components have a greater amplitude attenuation with increasing distance from the source, which allows them to record the signal from a smaller distance (nearby). Lower frequency (LF) components have a lower amplitude attenuation with increasing distance from the source, which allows them to record the signal from a higher distance (distant). The difference DIFF between LF and HF components mainly attenuates amplitudes from more distant regions.
**Figure 3** shows the attenuation of the ECG signal amplitude of ventricular activation, depending on the analyzed frequency band and on the distance between the electrode and activated myocardial cells. The attenuation with increasing distance has a significantly higher decay in higher-frequency envelopes than in lower-frequency envelopes. The left panel demonstrates the distance measurement between the body surface electrodes and the nearest epicardium. Magnetic resonance imaging was used to measure distances. The right panel shows the amplitude of the envelopes decreases for different frequency bands. The horizontal axis is the relative distance from the ventricles, and the vertical axis is the relative amplitude. Selected bands: LF 0.2-20 Hz, MF 20-80 Hz, HF 80-300, UHF1 300-500 Hz, and UHF2 800-1000 Hz. The results show a statistical evaluation of 130 subjects.

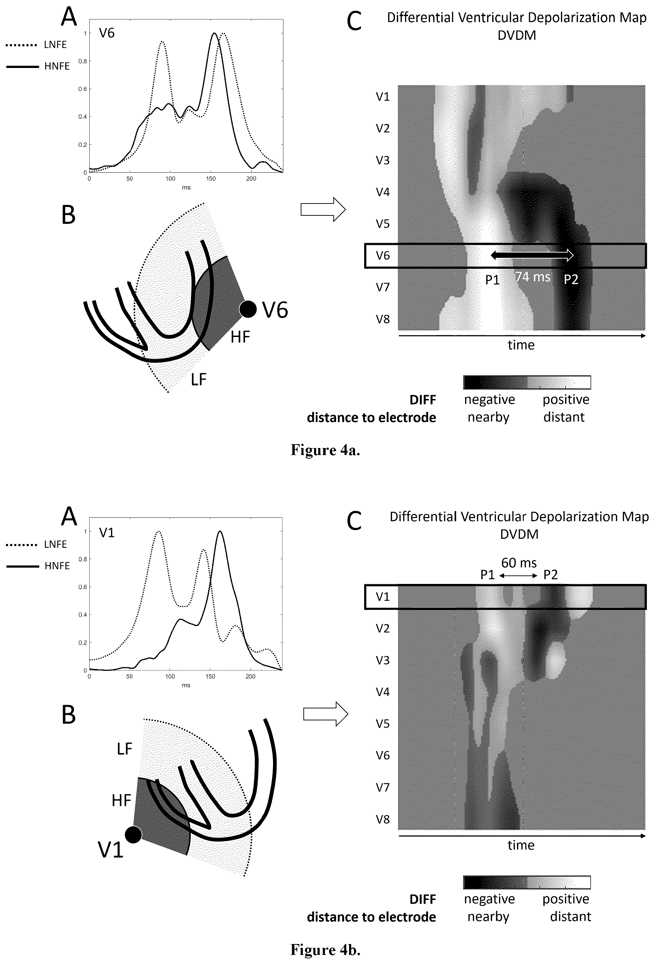

**Figure 4a** shows a Differential Ventricular Depolarization Map DVDM for a patient with the left bundle branch block pattern. A-normalized frequency envelopes LNFE and HFNE in lead V6. LNFE in the frequency band 20-150 Hz. HNFE in the frequency band 700-1000 Hz. B-schematic representation of visible areas for lower and higher frequencies. C-DVDM-differential ventricular depolarization map. The time in the range 120 ms before and 120 ms after the QRS annotation mark is plotted on the horizontal axis. The vertical axis defines the leads V1-V8. Light shades of gray identify areas farther from the electrode (distant), and dark shades of gray identify areas closer to the electrode (nearby). The light gray color in DVDM, lead V6, identifies activation of the septum and apex. Dark color identifies left ventricular free wall activation. In this case, the delay in the activation of the free wall of the left ventricle is 74 milliseconds. The time interval is measured between the maximum positive and negative difference DIFF or manually by cursors in lead V6.
**Figure 4b** shows the Differential Ventricular Depolarization Map DVDM for a patient with the right bundle branch block pattern. A-normalized frequency envelopes LNFE and HFNE in lead V1. LNFE in the frequency band 20-150 Hz. HNFE in the frequency band 700-1000 Hz. B-schematic representation of visible areas for lower and higher frequencies. C-DVDM-differential ventricular depolarization map. The time in the range 120 ms before and 120 ms after the QRS annotation mark is plotted on the horizontal axis. The vertical axis defines the lead V1-V8. Light shades of gray identify areas farther from the electrode (distant), and dark shades of gray identify areas closer to the electrode (nearby). The light gray color in DVDM, lead V1, identifies activation of the septum and apex. Dark color identifies right ventricular free wall activation. In this case, the delay in the activation of the free wall of the right ventricle is 60 milliseconds. The time interval is measured between the maximum positive and negative difference DIFF or manually by cursors in lead V1.

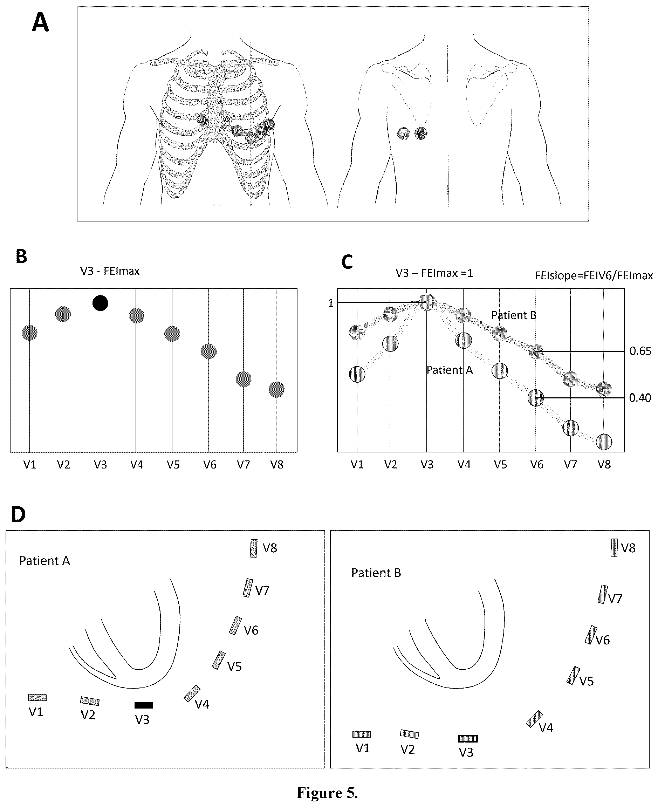

**Figure 5** shows a procedure for identification of heart distance from the body surface.
   A - example of electrode placement on the body surface.
   B - FEI computed in V1-V8 leads, FEI is here an integral of the average envelopes in the frequency band 700-1000 Hz. The lead V3 with the maximum value FEImax shows the location of the body surface where the heart is closest to the body surface.
   C - FEIslope is calculated, wherein FEIslope is defined as the proportion of FEI in a single lead to FEImax. FEIslope indicates the heart's distance from the body's surface. A lower FEIslope value of 0,40 means a higher drop in the FEI values relative to the maximal value. This indicates a smaller distance of the heart ventricles from the body surface. A higher FEIslope value of 0,65 means a small drop in the FEI values relative to the maximal value. This indicates a far distance of the heart ventricles from the body surface. A faster relative amplitude decrease in Patient A indicates a shorter distance between the heart and the chest electrodes in comparison with Patient B.
   D - The electrodes on the surface of the chest are at different distances from the heart. Patient A has electrodes near the heart, and patient B has electrodes further from the heart.

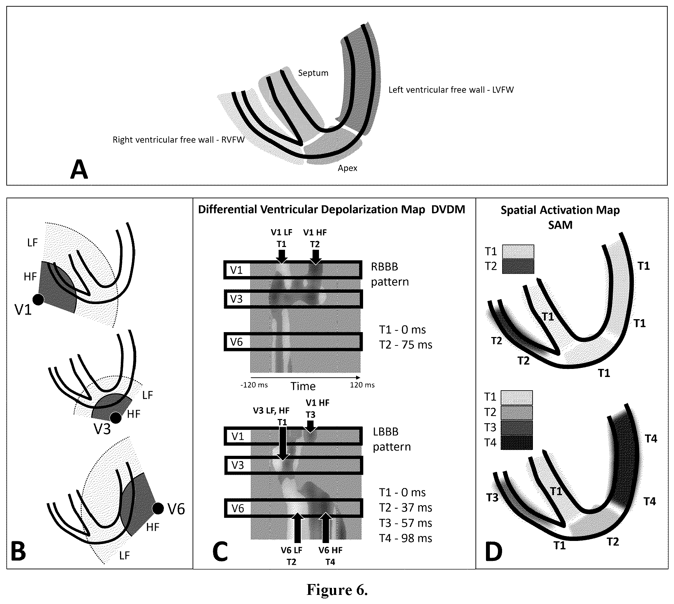

**Figure 6** shows a Spatial Activation Map (SAM) constructed as a projection of DVDM onto the geometrical interpretation of the ventricles. A - schematic designation of the segments in the ventricles - right ventricular free wall (RVFW), septum, apex, and left ventricular free wall. B - schematic interpretation of ventricular areas that record higher frequencies of HF and lower frequencies of LF in leads V1, V3, and V6. C - Differential Ventricular Depolarization Maps DVDM, in the upper part right bundle branch block (RBBB) patient, in the lower part left bundle branch block (LBBB) patient. Light shades of gray identify areas farther from the leads V1, V3, and V6, and dark shades of gray identify areas closer to the leads V1, V3, and V6 - marked by dark rectangles. T1, T2, T3, and T4 times were determined using cursors in the lightest and darkest regions of the DVDM for lead V1 in the RBBB subject and leads V1, V3, and V6 in the LBBB subject. Time T1 was set as the time of first activation and is therefore equal to zero. D - schematic projection of activation times into segments according to panel A. The local activation time is identified by the segment's color shade.

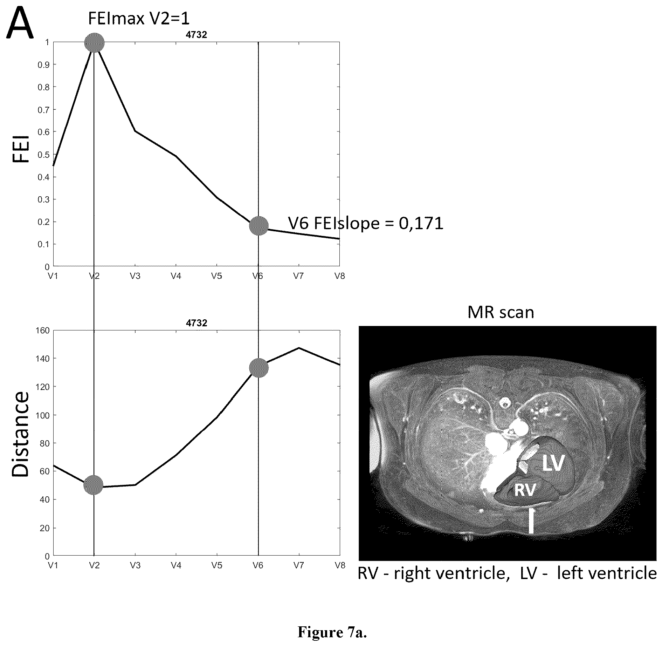

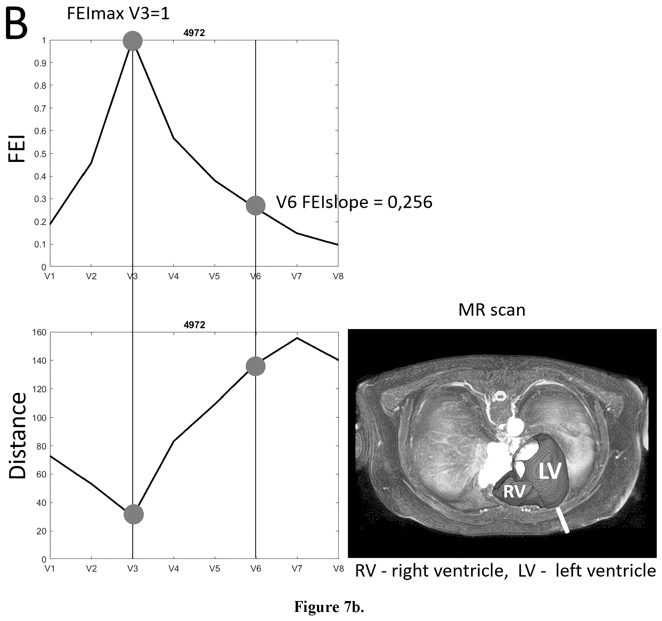

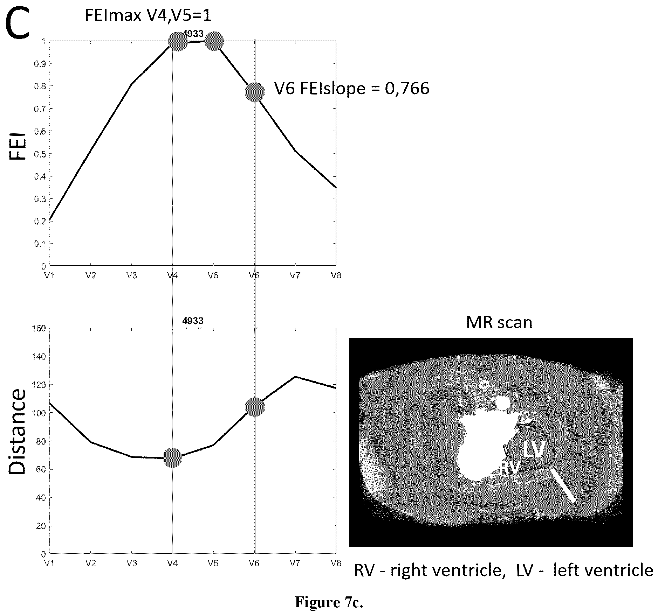

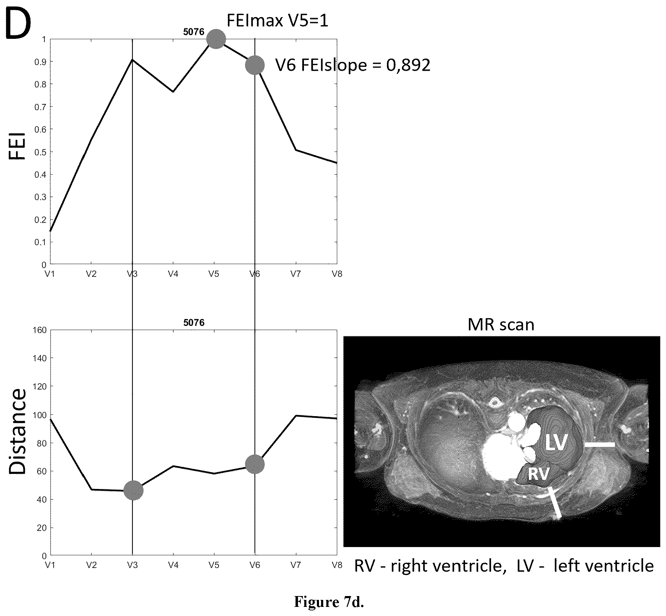

**Figure 7** shows examples of FEI and FEIslope parameters computation for different heart positions in the chest. The FEI graph shows the FEI parameter values for leads V1-V8 computed in the frequency range 700-1000 Hz. The maximum FEI value in this graph is normalized to 1. The vertical scale of the graph thus corresponds to the value of the FEIslope index. The Distance graph shows the body surface electrode distances to the epicardium for leads V1-V8 in millimeters.
   A - subject with a short heart-to-chest distance in leads V2 and V3 and long heart-to-chest distance in leads V6, V7 and V8. The smallest distance is in lead V2. A very low V6 FEIslope value indicates a relatively low V6 FEI value versus V2 FEI value.
   B - a subject with a very short heart-to-chest distance in lead V3 and long heart-to-chest distance in leads V6, V7, and V8. A very low V6 FEIslope value indicates a relatively low V6 FEI value versus V3 FEI value.
   C - subject with a long heart-to-chest distance and small differences between leads. The smallest distance is in leads V4 and V5. A very high V6 FEIslope value indicates a relatively high V6 FEI value versus V4 and V5 values FEI and small inter-lead differences.
   D - subject with small differences in heart-to-chest distances. The smallest distance is in leads V3, V5 and V6. A very high V6 FEIslope value indicates a relatively high V6 FEI value versus maximal values FEI and small inter-lead differences.

### Examples

The invention is further illustrated by examples of clinical application of the method according to the invention. The examples are based on the following ECG recording and processing configuration:
ECG signal was recorded with a sampling frequency of 5 kHz, a dynamic range of 26 bits (3 nV resolution), and a frequency range of 1.5 kHz. ECG data was collected over 0.5-5 minutes in a resting supine position with a standard 14-lead ECG setup.

For each precordial lead (eight ECG channels V1-V8), the amplitude envelopes of the signal were computed in the following frequency bands: 20-120, 25-125, 30-130, 35-135, 40-140, 45-145, 50-150, 700-800, 725-825, 750-850, 775-875, 800-900, 825-925, 850-950, 875-975, and 900-1000 Hz, using the Hilbert transform.

Combined median envelopes (ANFE) were calculated in the following manner:
- calculated envelopes were divided in each frequency range in each channel into QRS complex envelopes regions, wherein each QRS complex envelope comprises one annotated QRS complex;
- for each of the selected frequency ranges in each channel, a median envelope (FE) was computed as a median of the QRS complex envelopes in the frequency range;
- baseline correction for each median envelope (FE) by subtracting the mean value of an interval in which no QRS complex is present was computed, wherein the interval in which no QRS complex is present is an interval between the QRS complexes to remove noise background;
- each median envelope (FE) was normalized to obtain a normalized median envelope (NFE) in each frequency range of each channel, wherein the normalizing was performed by dividing the median envelope (FE) of the frequency range by its integral reached in the median envelope, in each frequency range and each channel separately, while the integral was calculated within an interval 120 ms before the annotation of the QRS complex and 120 ms after the annotation of the QRS complex;
- lower-frequency (LF) combined average envelope (LANFE) was calculated from normalized median envelopes NFE from lowest frequency ranges 20-120, 25-125, 30-130, 35-135, 40-140, 45-145, 50-150 Hz within each channel;
- higher-frequency (HF) combined average envelope (HANFE) was calculated from normalized median envelopes NFE from highest frequency ranges 700-800, 725-825, 750-850, 775-875, 800-900, 825-925, 850-950, 875-975, and 900-1000 Hz within each channel.

The difference (DIFF) was computed for each channel between the values LANFE and HANFE in the lower frequency range and in the higher frequency range, respectively, as DIFF equals LANFE minus HANFE. The method of DIFF calculation is shown in Figure 2.

A positive difference DIFF identifies ventricular electrical activation of distant heart ventricular regions, while a negative difference DIFF identifies ventricular electrical activation of nearby heart ventricular regions. This assumption is based on a study that analyzed the rate of decline of signal amplitude for different frequency bands on 130 subjects. Figure 3 shows how the signal amplitude decreases with increasing distance. There is a noticeable difference, especially between LF (0.2-20 Hz), MF (20-80 Hz) and UHF2 (800-1000 Hz) frequencies.

An integral FEI of the average envelopes was compared between channels, wherein the channel with the maximum value of the integral FEImax corresponds to the body surface location where the heart is closest to the body surface. FEIslope value was calculated as the proportion of the integral FEI value corresponding to the relevant frequency range and the relevant channel to FEImax that indicates the heart's distance from the body surface, wherein a lower FEIslope value means a higher drop in the FEI values relative to the maximal value, thus indicating a smaller distance of the heart ventricles from the body surface, whereas a higher FEIslope value means a small drop in the FEI values relative to the maximal value, thus indicating a far distance of the heart ventricles from the body surface. Figure 5 shows how the FEI and FEIslope values are interpreted and how they identify the closer or farther position of the heart from the surface of the chest.

The following examples show the relationship between these parameters during different types of pathologies:
**Case 1:** Patient with left bundle branch block (LBBB), spontaneous rhythm, R3081, Figure 4a.
   An example of the differential ventricular depolarization map DVDM in a patient with a delayed activation of the left ventricular free wall (LVFW). Dual activation is evident in lead V6. In DVDM, the first activation peak, P1, is light gray, and the second activation peak, P2, is dark gray. The light gray color P1 peak identifies areas where the normalized LANFE envelope has a higher amplitude than the normalized HANFE envelope. This means that the distant signal has a relatively higher intensity. This, in the case of the V6 electrode position near the LVFW, means activation in the region of the septum and apex. The second dark gray color P2 peak identifies areas where the normalized HANFE envelope has a higher amplitude than the normalized LANFE envelope. This means that the distant signal has a relatively lower intensity. This, in the case of the V6 electrode position near the LVFW, means activation in the LVFW region. The P1 and P2 peaks thus identify the different activation times of the septum and apical regions and LVFW. The time difference in this case is 74 ms, which defines the delay with which the LVFW is activated.
**Case 2:** Patient with right bundle branch block (RBBB), spontaneous rhythm, R3081, Figure 4b.
   An example of the differential ventricular depolarization map DVDM in a patient with a delayed activation of the right ventricular free wall (RVFW). Dual activation is evident in lead V1. In DVDM, the first activation peak P1 is light gray, and the second activation peak P2 is dark gray. The light gray color P1 peak identifies areas where the normalized LANFE envelope has a higher amplitude than the normalized HANFE envelope. This means that the distant signal has a relatively higher intensity. This, in the case of the V1 electrode position near the RVFW, means activation in the region of the septum and apex. The second dark gray color P2 peak identifies areas where the normalized HANFE envelope has a higher amplitude than the normalized LANFE envelope. This means that the distant signal has a relatively lower intensity. This, in the case of the V1 electrode position near the RVFW, means activation in the region of RVFW. The P1 and P2 peaks thus identify the different times of activation of the septum and apical regions and RVFW. The time difference in this case is 60 ms, which defines the delay with which the RVFW is activated.
**Case 3:** Two subjects with RBBB ( R0281) and LBBB (R0117), Figure 6. The example shows the transformation of DVDM into a spatial activation map SAM. The transformation is based on the information provided by the DVDM and the knowledge of the position of the specific electrode. The DVDM map includes electrodes V1 - V8 on the vertical axis. At the same time, electrodes V1, V3, and V6 are highlighted, and activation times T1, T2, and T3 are identified on them.

In the case of an RBBB patient, the T1 time in lead V1 corresponds to more distant activation. At the time T1, there is one activation peak in the other leads. The T1 identifies the time of simultaneous activation of the septum, apex, and LVFW. A T2 peak in lead V1 corresponds to closer activation and points to RVFW. In this case, the RVFW activation delay is 75 ms. For better clarity, this information is shown schematically in SAM, right panel top. Individual segments (Figure 6a) are assigned a color corresponding to the activation time.

In the case of an LBBB patient, the first activation time, T1, in lead V3 corresponds to both close and more distant activation. In contrast, the T2 time in lead V6 indicates distal activation (septal), and the T4 time indicates close activation (LVFW). Time T3 in lead V1 indicates close activation (RVFW). Individual activation times localized in ventricular segments are transferred to SAM, right panel bottom.

**Case 4:** Four subjects with different heart positions in the chest cavity, Figure 7. The case shows examples of FEI and FEIslope parameter computation for different heart positions in the chest. It demonstrates the relationship between the high value of the FEIslope parameter and the high distance between the heart ventricles and electrodes on the body surface. At the same time, the position of the maximum of the FEI parameter in V leads indicates the placement of the heart in the chest relative to the position of the electrodes.

Figure 7A - represents a subject (R4732) with a short heart-to-chest distance in leads V2 and V3 and long heart-to-chest distance in leads V6, V7, and V8. The smallest distance is in lead V2 - indicated by the white line in the MR scan. A very low V6 FEIslope value means a relatively low V6 FEI value versus V2 FEImax value. This high difference between V6 FEI and V2 FEImax indicates the position of the heart near the chest. The heart is close to the front of the chest and far from the back.

Figure 7B - a subject (R4972) with a short heart-to-chest distance in lead V3 and long heart-to-chest distance in leads V6, V7, and V8. The smallest distance is in lead V3 - indicated by the white line in the MR scan. A very low V6 FEIslope value indicates a relatively low V6 FEI value versus V3 FEI value. A low V6 FEIslope value means a low distance of V leads from ventricles. The heart is close to the front of the chest and far from the back.

Both figures 7A and 7B show the close position of the heart from the front side of the chest. The difference in the figures lies in the position of the FEI maximum in lead V2 or lead V3. This indicates a different rotation of the heart in the chest relative to leads V leads.

Figure 7C - a subject (R4933) with a long heart-to-chest distance and small differences between leads. The smallest distance is in leads V4 and V5. A very high V6 FEIslope value indicates a relatively high V6 FEI value versus V4 and V5 values FEI. A very high V6 FEIslope value indicates a higher and comparable distance of V leads from ventricles.

Figure 7D shows a subject (R5076) with small differences in heart-to-chest distances. The smallest distance is in leads V3, V5, and V6. This indicates an abnormal position of the heart relative to the V leads. A very high V6 FEIslope value indicates a higher distance of V leads from ventricles and simultaneously atypical position of the heart ventricular segments on both the front and back sides.

## Claims

1. A method of processing an electrocardiogram, which comprises the following steps:
- measuring the electrocardiogram comprising at least two channels and sensing a separate electrocardiogram signal on each of the at least two channels; wherein the measuring step is carried out by an apparatus comprising at least two sensors measuring the electrocardiogram signal, wherein the outputs of the at least two sensors are connected to an input of one or more analogue amplifiers, and an output of the said one or more analogue amplifiers is connected to an input of one or more analogue signal to digital signal converters, and an output of the one or more analogue signal to digital signal converters is connected to a processing unit, wherein the at least two sensors, the one or more analogue amplifiers, and the one or more analogue signal to digital signal converters have the transmission bandwidth of at least 0.2 kHz,
- and performing the following steps in a processing unit, comprising an input connected to the output of the analogue to digital signal converters and an output connected to at least one imaging unit:
- selecting at least two, preferably non-overlapping, frequency ranges of the signal in each of the said at least two channels;
- calculating an amplitude or power envelope of the signal in each selected frequency range in each channel;
- dividing the calculated envelope of the signal in each frequency range in each channel into QRS complex envelopes, wherein each QRS complex envelope comprises one annotated QRS complex;
- computing, for each of the selected frequency ranges in each channel, an average envelope or a median envelope, FE, as an average or a median of the QRS complex envelopes in the frequency range;
- optionally performing baseline correction for each average envelope or each median envelope by subtracting the mean or median value of an interval in which no QRS complex is present, wherein the interval in which no QRS complex is present is an interval between the QRS complexes, to remove noise background;
- normalizing each average or median envelope, FE, optionally after its baseline correction, to obtain a normalized average envelope or a normalized median envelope, NFE, in each frequency range of each channel; wherein the normalizing is performed by dividing the average envelope or the median envelope, FE, of the frequency range by its integral or by a maximal value reached in the average envelope or in the median envelope, in each frequency range and each channel separately, while the integral or the maximal value is calculated within an interval of at least 30 ms before the annotation of the QRS complex and at least 30 ms after the annotation of the QRS complex;
- if more than two frequency ranges are selected in each channel, a combined average envelope or a combined median envelope, ANFE, is calculated from normalized average envelopes or normalized median envelopes, NFE, from at least two lowest frequency ranges and/or from at least two highest frequency ranges within each channel, wherein each frequency range is included in only one combined average envelope or combined median envelope, ANFE, and the calculation results in two combined average envelopes or combined median envelopes, ANFE;
- calculating, for each channel, the difference between the values FE, NFE and/or ANFE in the lower frequency range and the values FE, NFE and/or ANFE in the higher frequency range; wherein
- a positive difference between the values FE, NFE and/or ANFE in the lower frequency range and the values FE, NFE and/or ANFE in the higher frequency range identifies ventricular electrical activation of distant heart ventricular regions;
- a negative difference between the values FE, NFE and/or ANFE in the lower frequency range and the values FE, NFE and/or ANFE in the higher frequency range identifies ventricular electrical activation of nearby heart ventricular regions.

2. Method according to claim 1, further comprising a step of constructing a differential ventricular depolarization map, **DVDM,** as a matrix wherein each row of differential ventricular depolarization matrix is represented by a difference between LFE and HFE, LNFE and HNFE or LANFE and HANFE for one of the said at least two ECG channels, and the columns correspond to time intervals, and then the positive values in each differential ventricular depolarization matrix row are assigned a first colour and negative values in each differential ventricular depolarization matrix row are assigned a second colour, wherein the first colour in DVDM identifies ventricular electrical activation of distant heart ventricular regions at the respective time point or time interval, and the second colour identifies ventricular electrical activation of nearby heart ventricular regions at the respective time point or time interval; and a step of imaging the matrix by the at least one imaging unit.

3. Method according to claim 1, wherein the method further comprises a step of comparison of an integral **FEI** of the average envelopes or median envelopes between channels, wherein the channel with the maximum value of the integral **FEImax** corresponds to the body surface location where the heart is closest to the body surface, and
a subsequent step of calculating a value FEIslope defined as the proportion of the integral FEI value corresponding to the relevant frequency range and the relevant channel to FEImax, wherein the FEImax indicates the heart's distance from the body surface, wherein a lower **FEIslope** value means a higher drop in the FEI values relative to the maximal value, thus indicating a smaller distance of the heart ventricles from the body surface, whereas a higher FEIslope value means a small drop in the FEI values relative to the maximal value, thus indicating a far distance of the heart ventricles from the body surface.

4. Method according to claim 2, wherein the method further comprises a step of construction of a spatial activation map **SAM,** wherein the spatial activation map is constructed as a projection of the differential ventricular depolarization map to geometrical interpretation of the ventricles, wherein the first colour identifies ventricular electrical activation of the distant ventricular septal region in all V1-V8 leads, and the second colour identifies ventricular electrical activation of the nearby ventricular regions, which are: in leads V1-V2 of the free wall of the right ventricle, in leads V3-V4 of the apical region, and in leads V5-V8 of the free wall of the left ventricle; and a step of imaging the spatial activation map by the at least one imaging unit.

## Patentansprüche

1. Verfahren zur Verarbeitung eines Elektrokardiogramms, umfassend die folgenden Schritte:
- Messen des Elektrokardiogramms, das mindestens zwei Kanäle umfasst, und Erfassen eines separaten Elektrokardiogrammsignals auf jedem der mindestens zwei Kanäle; wobei der Messschritt mittels einer Vorrichtung durchgeführt wird, die mindestens zwei das Elektrokardiogrammsignal messende Sensoren umfasst, wobei die Ausgänge der mindestens zwei Sensoren mit einem Eingang eines oder mehrerer analoger Verstärker verbunden sind, und ein Ausgang des einen oder der mehreren analogen Verstärker mit einem Eingang eines oder mehrerer Analog-Digital-Signalwandler verbunden ist, und ein Ausgang des einen oder der mehreren Analog-Digital-Signalwandler mit einer Verarbeitungseinheit verbunden ist, wobei die mindestens zwei Sensoren, der eine oder die mehreren analogen Verstärker und der eine oder die mehreren Analog-Digital-Signalwandler eine Übertragungsbandbreite von mindestens 0,2 kHz aufweisen,
- und Durchführen der folgenden Schritte in einer Verarbeitungseinheit, die einen mit dem Ausgang der Analog-Digital-Signalwandler verbundenen Eingang und einen mit mindestens einer Bildgebungseinheit verbundenen Ausgang umfasst:
- Auswählen von mindestens zwei, vorzugsweise nicht überlappenden, Frequenzbereichen des Signals in jedem der genannten mindestens zwei Kanäle;
- Berechnen einer Amplituden- oder Leistungshüllkurve des Signals in jedem ausgewählten Frequenzbereich jedes Kanals;
- Aufteilen der berechneten Hüllkurve des Signals in jedem Frequenzbereich jedes Kanals in QRS-Komplex-Hüllkurven, wobei jede QRS-Komplex-Hüllkurve einen annotierten QRS-Komplex umfasst;
- Berechnen, für jeden der ausgewählten Frequenzbereiche jedes Kanals, einer mittleren Hüllkurve oder einer Medianhüllkurve, FE, als Mittelwert oder Median der QRS-Komplex-Hüllkurven in dem Frequenzbereich;
- gegebenenfalls Durchführen einer Basislinienkorrektur für jede mittlere Hüllkurve oder jede Medianhüllkurve durch Subtrahieren des Mittel- oder Medianwerts eines Intervalls, in dem kein QRS-Komplex vorhanden ist, wobei das Intervall, in dem kein QRS-Komplex vorhanden ist, ein Intervall zwischen den QRS-Komplexen ist, um das Hintergrundrauschen zu entfernen;
- Normalisieren jeder mittleren oder Medianhüllkurve, FE, gegebenenfalls nach deren Basislinienkorrektur, um eine normalisierte mittlere Hüllkurve oder eine normalisierte Medianhüllkurve, NFE, in jedem Frequenzbereich jedes Kanals zu erhalten; wobei das Normalisieren durch Division der mittleren Hüllkurve oder der Medianhüllkurve, FE, des Frequenzbereichs durch ihr Integral oder durch einen in der mittleren Hüllkurve oder in der Medianhüllkurve erreichten Maximalwert erfolgt, und zwar in jedem Frequenzbereich und jedem Kanal separat, wobei das Integral oder der Maximalwert innerhalb eines Intervalls von mindestens 30 ms vor der Annotation des QRS-Komplexes und mindestens 30 ms nach der Annotation des QRS-Komplexes berechnet wird;
- wenn in jedem Kanal mehr als zwei Frequenzbereiche ausgewählt werden, wird eine kombinierte mittlere Hüllkurve oder eine kombinierte Medianhüllkurve, ANFE, aus normalisierten mittleren Hüllkurven oder normalisierten Medianhüllkurven, NFE, von mindestens zwei niedrigsten Frequenzbereichen und/oder von mindestens zwei höchsten Frequenzbereichen innerhalb jedes Kanals berechnet, wobei jeder Frequenzbereich nur in einer kombinierten mittleren Hüllkurve oder kombinierten Medianhüllkurve, ANFE, enthalten ist, und die Berechnung zu zwei kombinierten mittleren Hüllkurven oder kombinierten Medianhüllkurven, ANFE, führt;
- Berechnen, für jeden Kanal, der Differenz zwischen den Werten FE, NFE und/oder ANFE in dem niedrigeren Frequenzbereich und den Werten FE, NFE und/oder ANFE in dem höheren Frequenzbereich;
wobei
- eine positive Differenz zwischen den Werten FE, NFE und/oder ANFE in dem niedrigeren Frequenzbereich und den Werten FE, NFE und/oder ANFE in dem höheren Frequenzbereich eine ventrikuläre elektrische Aktivierung entfernter ventrikulärer Herzregionen kennzeichnet;
- eine negative Differenz zwischen den Werten FE, NFE und/oder ANFE in dem niedrigeren Frequenzbereich und den Werten FE, NFE und/oder ANFE in dem höheren Frequenzbereich eine ventrikuläre elektrische Aktivierung naher ventrikulärer Herzregionen kennzeichnet.

2. Verfahren nach Anspruch 1, ferner umfassend einen Schritt des Konstruierens einer differenziellen ventrikulären Depolarisations-Karte, DVDM, als Matrix, wobei jede Zeile der differenziellen ventrikulären Depolarisations-Matrix durch eine Differenz zwischen LFE und HFE, LNFE und HNFE oder LANFE und HANFE für einen der genannten mindestens zwei EKG-Kanäle dargestellt wird, und die Spalten Zeitintervallen entsprechen, und wobei anschließend den positiven Werten in jeder Zeile der differenziellen ventrikulären Depolarisations-Matrix eine erste Farbe und den negativen Werten in jeder Zeile der differenziellen ventrikulären Depolarisations-Matrix eine zweite Farbe zugewiesen wird, wobei die erste Farbe in der DVDM eine ventrikuläre elektrische Aktivierung entfernter ventrikulärer Herzregionen zum jeweiligen Zeitpunkt oder Zeitintervall kennzeichnet, und die zweite Farbe eine ventrikuläre elektrische Aktivierung naher ventrikulärer Herzregionen zum jeweiligen Zeitpunkt oder Zeitintervall kennzeichnet; und einen Schritt des Abbildens der Matrix durch die mindestens eine Bildgebungseinheit.

3. Verfahren nach Anspruch 1, wobei das Verfahren ferner einen Schritt des Vergleichens eines Integrals FEI der mittleren Hüllkurven oder Medianhüllkurven zwischen den Kanälen umfasst, wobei der Kanal mit dem Maximalwert des Integrals FEImax der Körperoberflächenstelle entspricht, an der das Herz der Körperoberfläche am nächsten liegt, und
einen nachfolgenden Schritt des Berechnens eines Werts FEIslope, definiert als das Verhältnis des Integralwerts FEI, der dem betreffenden Frequenzbereich und dem betreffenden Kanal entspricht, zu FEImax, wobei FEImax den Abstand des Herzens von der Körperoberfläche angibt, wobei ein niedrigerer FEIslope-Wert einen stärkeren Abfall der FEI-Werte relativ zum Maximalwert bedeutet und somit einen geringeren Abstand der Herzventrikel von der Körperoberfläche anzeigt, und wobei ein höherer FEIslope-Wert einen geringen Abfall der FEI-Werte relativ zum Maximalwert bedeutet und somit einen großen Abstand der Herzventrikel von der Körperoberfläche anzeigt.

4. Verfahren nach Anspruch 2, wobei das Verfahren ferner einen Schritt des Konstruierens einer räumlichen Aktivierungs-Karte SAM umfasst, wobei die räumliche Aktivierungskarte als Projektion der differenziellen ventrikulären Depolarisations-Karte auf eine geometrische Interpretation der Ventrikel konstruiert wird, wobei die erste Farbe eine ventrikuläre elektrische Aktivierung der entfernten ventrikulären Septumregion in allen Ableitungen V1 bis V8 kennzeichnet, und die zweite Farbe eine ventrikuläre elektrische Aktivierung der nahen ventrikulären Regionen kennzeichnet, welche sind: in den Ableitungen V1-V2 die freie Wand des rechten Ventrikels, in den Ableitungen V3-V4 die apikale Region, und in den Ableitungen V5-V8 die freie Wand des linken Ventrikels; und einen Schritt des Abbildens der räumlichen Aktivierungs-Karte durch die mindestens eine Bildgebungseinheit.

## Revendications

1. Procédé de traitement d'un électrocardiogramme, comprenant les étapes suivantes :
- la mesure de l'électrocardiogramme comprenant au moins deux canaux et la détection d'un signal d'électrocardiogramme distinct sur chacun des au moins deux canaux ; l'étape de mesure étant réalisée au moyen d'un appareil comprenant au moins deux capteurs mesurant le signal d'électrocardiogramme, les sorties des au moins deux capteurs étant connectées à une entrée d'un ou plusieurs amplificateurs analogiques, et une sortie dudit ou desdits amplificateurs analogiques étant connectée à une entrée d'un ou plusieurs convertisseurs de signal analogique en signal numérique, et une sortie du ou des convertisseurs de signal analogique en signal numérique étant connectée à une unité de traitement, les au moins deux capteurs, le ou les amplificateurs analogiques et le ou les convertisseurs de signal analogique en signal numérique présentant une bande passante de transmission d'au moins 0,2 kHz,
- et la réalisation des étapes suivantes dans une unité de traitement, comprenant une entrée connectée à la sortie des convertisseurs de signal analogique en signal numérique et une sortie connectée à au moins une unité d'imagerie :
- la sélection d'au moins deux plages de fréquences, de préférence non chevauchantes, du signal dans chacun desdits au moins deux canaux ;
- le calcul d'une enveloppe d'amplitude ou de puissance du signal dans chaque plage de fréquences sélectionnée de chaque canal ;
- la division de l'enveloppe calculée du signal dans chaque plage de fréquences de chaque canal en enveloppes de complexe QRS, chaque enveloppe de complexe QRS comprenant un complexe QRS annoté ;
- le calcul, pour chacune des plages de fréquences sélectionnées de chaque canal, d'une enveloppe moyenne ou d'une enveloppe médiane, FE, en tant que moyenne ou médiane des enveloppes de complexe QRS dans la plage de fréquences ;
- éventuellement la réalisation d'une correction de la ligne de base pour chaque enveloppe moyenne ou chaque enveloppe médiane par soustraction de la valeur moyenne ou médiane d'un intervalle dans lequel aucun complexe QRS n'est présent, ledit intervalle dans lequel aucun complexe QRS n'est présent étant un intervalle entre les complexes QRS, afin d'éliminer le bruit de fond ;
- la normalisation de chaque enveloppe moyenne ou médiane, FE, éventuellement après sa correction de ligne de base, pour obtenir une enveloppe moyenne normalisée ou une enveloppe médiane normalisée, NFE, dans chaque plage de fréquences de chaque canal ; la normalisation étant réalisée en divisant l'enveloppe moyenne ou l'enveloppe médiane, FE, de la plage de fréquences par son intégrale ou par une valeur maximale atteinte dans l'enveloppe moyenne ou dans l'enveloppe médiane, dans chaque plage de fréquences et chaque canal séparément, l'intégrale ou la valeur maximale étant calculée dans un intervalle d'au moins 30 ms avant l'annotation du complexe QRS et d'au moins 30 ms après l'annotation du complexe QRS ;
- si plus de deux plages de fréquences sont sélectionnées dans chaque canal, une enveloppe moyenne combinée ou une enveloppe médiane combinée, ANFE, est calculée à partir des enveloppes moyennes normalisées ou des enveloppes médianes normalisées, NFE, d'au moins deux des plages de fréquences les plus basses et/ou d'au moins deux des plages de fréquences les plus hautes au sein de chaque canal, chaque plage de fréquences n'étant incluse que dans une seule enveloppe moyenne combinée ou enveloppe médiane combinée, ANFE, et le calcul aboutissant à deux enveloppes moyennes combinées ou enveloppes médianes combinées, ANFE ;
- le calcul, pour chaque canal, de la différence entre les valeurs FE, NFE et/ou ANFE dans la plage de fréquences inférieure et les valeurs FE, NFE et/ou ANFE dans la plage de fréquences supérieure ;
où
- une différence positive entre les valeurs FE, NFE et/ou ANFE dans la plage de fréquences inférieure et les valeurs FE, NFE et/ou ANFE dans la plage de fréquences supérieure identifie une activation électrique ventriculaire de régions ventriculaires cardiaques distantes ;
- une différence négative entre les valeurs FE, NFE et/ou ANFE dans la plage de fréquences inférieure et les valeurs FE, NFE et/ou ANFE dans la plage de fréquences supérieure identifie une activation électrique ventriculaire de régions ventriculaires cardiaques proches.

2. Procédé selon la revendication 1, comprenant en outre une étape de construction d'une carte de dépolarisation ventriculaire différentielle, DVDM, sous forme de matrice, dans laquelle chaque ligne de la matrice de dépolarisation ventriculaire différentielle est représentée par une différence entre LFE et HFE, LNFE et HNFE, ou LANFE et HANFE pour l'un desdits au moins deux canaux ECG, et les colonnes correspondent à des intervalles de temps, puis les valeurs positives de chaque ligne de la matrice de dépolarisation ventriculaire différentielle se voient attribuer une première couleur et les valeurs négatives de chaque ligne de la matrice de dépolarisation ventriculaire différentielle se voient attribuer une deuxième couleur, la première couleur dans la DVDM identifiant une activation électrique ventriculaire de régions ventriculaires cardiaques distantes au point temporel ou à l'intervalle de temps respectif, et la deuxième couleur identifiant une activation électrique ventriculaire de régions ventriculaires cardiaques proches au point temporel ou à l'intervalle de temps respectif ; et une étape de représentation de la matrice par ladite au moins une unité d'imagerie.

3. Procédé selon la revendication 1, où le procédé comprend en outre une étape de comparaison d'une intégrale FEI des enveloppes moyennes ou des enveloppes médianes entre les canaux, le canal présentant la valeur maximale de l'intégrale FEImax correspondant à l'emplacement de la surface corporelle où le cœur est le plus proche de la surface corporelle, et
une étape ultérieure de calcul d'une valeur FEIslope définie comme la proportion de la valeur de l'intégrale FEI correspondant à la plage de fréquences et au canal concernés par rapport à FEImax, la FEImax indiquant la distance du cœur par rapport à la surface corporelle, une valeur FEIslope plus faible signifiant une chute plus importante des valeurs FEI par rapport à la valeur maximale, indiquant ainsi une distance plus faible des ventricules cardiaques par rapport à la surface corporelle, tandis qu'une valeur FEIslope plus élevée signifie une faible chute des valeurs FEI par rapport à la valeur maximale, indiquant ainsi une distance importante des ventricules cardiaques par rapport à la surface corporelle.

4. Procédé selon la revendication 2, où le procédé comprend en outre une étape de construction d'une carte d'activation spatiale SAM, la carte d'activation spatiale étant construite en tant que projection de la carte de dépolarisation ventriculaire différentielle sur une interprétation géométrique des ventricules, la première couleur identifiant une activation électrique ventriculaire de la région septale ventriculaire distante dans toutes les dérivations V1 à V8, et la deuxième couleur identifiant une activation électrique ventriculaire des régions ventriculaires proches, à savoir : dans les dérivations V1-V2 de la paroi libre du ventricule droit, dans les dérivations V3-V4 de la région apicale, et dans les dérivations V5-V8 de la paroi libre du ventricule gauche ; et une étape de représentation de la carte d'activation spatiale par ladite au moins une unité d'imagerie.
